# EUROPÄISCHE PATENTANMELDUNG

(11) **EP 1 754 486 A2**
(43) Veröffentlichungstag der Anmeldung: **21.02.2007**
(21) Anmeldenummer: 06118997.3
(22) Anmeldetag: 16.08.2006
(51) Int. Cl.: A61K 38/00

(54) **Pharmazeutischer Wirkstoff zur Behandlung von Hepatitis, Borreliose und Multipler Sklerose**

(30) Priorität: 16.08.2005 DE 102005038768; 28.11.2005 DE 102005056513
(71) Anmelder: TOXIMED GmbH, 80331 München (DE)
(72) Erfinder: Weickmann, Dirk, 80339, München (DE)
(74) Vertreter: Behnisch, Werner

(57) **Zusammenfassung**

Die vorliegende Erfindung offenbart einen pharmazeutischer Wirkstoff zur Behandlung von Hepatitis A, Hepatitis C, Borreliose und/oder Multipler Sklerose, enthaltend in einer pharmazeutisch wirksamen Menge zumindest ungereinigtes Drüsensekret, gewonnen von Tieren der Klasse Diplopoda. Insbesondere offenbart die Erfindung einen pharmazeutischer Wirkstoff zur Behandlung der oben genannten Krankheiten, insbesondere von Borreliose, enthaltend in einer pharmazeutisch wirksamen Menge ein Drüsensekret eines Diplopoden, insbesondere eines afrikanischen Diplopoden, zumindest ein Peptidtoxin, und zumindest eine hierzu antagonistisch oder synergistisch wirkende Substanz und/oder eine Durchdringungssubstanz, wobei das Peptidtoxin aus dem Gift von Spinnen, Skorpionen oder Skolopendern stammt. Daneben wird auch ein Verfahren zu Herstellung des pharmazeutischen Wirkstoff offenbart.

## Beschreibung

Für die zum Erhalt des Lebens dienende Aufnahme von Nahrungsmitteln ist jedes Lebewesen auf das Angebot aus dem erreichbaren Pflanzen- und Tierreich angewiesen. Doch hierbei ist nicht alles ohne Gefahr zum Verzehr geeignet.
Viele Pflanzen und Tiere verwenden zum Schutz ihres eigenen Lebens und zum eigenen Nahrungserwerb, auf ihren speziellen Organismus und seine besonderen Bedürfnisse abgestimmte, so genannte biogene Gifte. Diese biogenen Gifte haben im Laufe langer Entwicklungszeiträume ihren Platz gefunden im Zusammenspiel der verschiedenen Arten von Leben.
Deshalb erkennt auch heute noch jedes erwachsene Wildtier gefährliche Pflanzen und giftige Tiere seiner natürlichen Umgebung.
Dabei können Pflanzen oder Tiere durch die Produktion von Giftstoffen primär giftig wirken oder erst durch die Aufnahme toxischer Substanzen aus der belebten oder unbelebten Umwelt sekundäre Toxizität erhalten.

Die Nutzung dieser biogenen Gifte begann in der Geschichte der Menschheit schon in der Urzeit als sie zur Erlegung von Beutetieren mit vergifteten Waffen diente.
Zur gefahrlosen Anwendung dieser Gifte waren jedoch von Anfang an gewisse Grundkenntnisse über deren Behandlung und Wirksamkeit erforderlich.
Die weiter durchgeführten Versuche, die Zusammensetzung des chemischen Aufbaus biogener Gifte zu entschlüsseln, führten später zur gezielten Suche bestimmter Wirkstoffe als eigentliche Verursacher beobachteter Wirkungen.

Insbesondere nach der von Paracelsus (1493 - 1541) erhobenen Forderung, die Wirkstoffe von Arzneipflanzen zu isolieren, die zur Entwicklung der latrochemie, also der Chemie hinsichtlich ihres ärztlichen Anwendungsbereichs, beitrug, dürften diese Bemühungen verstärkt haben. Vor allem die Kunst des Destillierens von Stoffen wurde in den Dienst der Forschung gestellt und lieferte eine Vielzahl ätherischer Öle und flüchtiger Stoffe. Aber für die Isolierung anderer Wirkstoffe oder gar für deren chemische Aufschlüsselung waren die damals bekannten Methoden unzureichend.

Erst zu Beginn des 19. Jahrhunderts war die Entwicklung der technischen Fertigkeiten in der Chemie weit genug fortgeschritten, die Ära der Isolierung von reinen Wirkstoffen aus biologischem Material einzuleiten.
Zunächst nutzte man, zur Abtrennung der gesuchten Wirkstoffe von den Begleitstoffen, die Unterschiede in der Löslichkeit der untersuchten Substanzen in verschiedenen Lösungsmitteln. Beobachtet wurden hierbei, zum Beispiel mit Fällungsmitten, die Unterschiede im Verteilungsverhalten zwischen zwei nicht mischbaren flüssigen Phasen, in der Flüchtigkeit und in der chemischen Reaktivität,

Einen gewaltigen Aufschwung in der Trenntechnik, dem Weg zur Ermittlung von Wirkstoffen zur Bekämpfung von Krankheiten, machte die Entwicklung chromatographischer Verfahren in der Mitte des 20. Jahrhunderts möglich. Ausgehend von der Verteilung zwischen einer mobilen und einer stationären flüssigen Phase, von der Adsorption, den Molekülsiebeffekten, dem Ionenaustausch, der Affinität (insbesondere von Proteinen) zu bestimmten chemischen Verbindungen (z.B. Enzymsubstraten) und der Beweglichkeit geladener Moleküle im elektrischen Feld, wurde eine Vielzahl neuer Trenntechniken entwickelt

Derzeit werden Tumore als die gefährlichsten und gefürchtetsten Krankheiten unserer Zeit auf eine sehr radikale und wenig umweltschonende Weise bekämpft. Als einfache, kennzeichnende Schlagworte können hier gelten:
Stahl, Strahl und Chemotherapie.
Das bedeutet einmal, dass Tumore, falls einigermaßen erreichbar, im Prinzip mit dem Stahl eines Messers herausgeschnitten, durch eine breitgefächerte Bestrahlung verbrannt, oder über eine sogenannte Chemotherapie mit auch gesunde Zellen angreifenden, aggressiven Zytostatika zerstört werden.
Sowohl bei normalen Behandlungen mit dem Skalpell als auch mit ionisierender Strahlung ist eine räumliche Begrenzung des Operationsgebiets nicht möglich. Es werden zwangsläufig auch gesunde Körperzellen vernichtet. Die unerwünschten Nebenwirkungen der Chemotherapie sind allgemein bekannt.
Im Gegensatz hierzu wurde aber auch versucht, eine Krebstherapie, die ihren Namen verdient, auf subtilere Weise zu ermöglichen. Zu diesem Zweck wurde auf den reichen Schatz der Natur zurückgegriffen.
Es werden hierzu unter anderem viele, aus giftigen Lebewesen isolierte, stark wirksame Stoffe in therapeutischen Dosen als Arzneistoffe genutzt.

So ist aus der DE 199 61 141 A1 ein pharmazeutischer Wirkstoff bekannt, bei dem gefunden wurde, dass Bestandteile der Spinnengifte von Spinnen der Familie Sicarildae zur Behandlung von Tumorerkrankungen verwendet werden können.
Es werden hierbei in der Hauptsache ein Peptidtoxin aus dem Gift dieser Spinnenart, eine weitere aus dem Gift gewonnene, antagonistisch wirkende Substanz und/oder eine Kombination dieser Bestandteile medizinisch genutzt.
Es kann dieser Wirkstoff zur Behandlung von Tumorerkrankungen sowie parallel bzw. unterstützend zu Tumoroperationen eingesetzt werden und Rest-Tumorgewebe zerstört werden. Bei der Therapie können genetisch veränderte Körperzellen (Tumorzellen) zerstört werden, da der betreffende Wirkstoff die veränderte Oberflächenstruktur solcher Zellen erkennt und komplikationsfrei abtötet.
Der Gesamtgiftgehalt dieser Spinnenart, sozusagen ein Cocktail verschiedener Substanzen, ist auf Grund seiner bereits in geringen Dosen letalen Wirkung nicht pharmazeutisch einsetzbar.

Dieser bekannte Wirkstoff wirkt jedoch in vivo in keiner Kombination bei der Bekämpfung anderer Krankheiten wie zum Beispiel der Borreliose

In der Folgezeit wurden zahlreiche Wirkstoffe gegen verschiedenste Tumorarten und andere Krankheiten auf der Basis biogener Gifte und anderer biogener Wirkstoffe entwickelt.
Für eine großtechnische Anwendung dieser Wirkstoffe werden jedoch große Mengen benötigt, die im Hinblick auf die geringen Mengen an Grundstoffen, die von den einzelnen Tierarten gewonnen werden können, nicht zu realisieren sind.

Hepatitis C, Borreliose und Multiple Sklerose werden im Folgenden näher beschrieben:

### Hepatitis A

Hepatitis A ist eine Entzündung der Leber. Die Erkrankung wird durch ein Virus, das Hepatitis A-Virus (HAV), verursacht. Dies wird meist durch Lebensmittel übertragen, die mit Kotrückständen verunreinigt sind. Die Infektion kommt vor allem in Südeuropa und Südosteuropa, Afrika, Asien, sowie in Südamerika und Mittelamerika vor. Etwa die Hälfte aller Hepatitis A-Fälle, die in Deutschland auftreten, betreffen Touristen aus südlichen Reiseländern. Die andere Hälfte wird häufig in Gemeinschaftseinrichtungen, wie z.B. in Kindergärten, übertragen.
Der Zeitabstand zwischen der eigentlichen Infektion und dem Ausbruch der Erkrankung (Inkubationszeit) beträgt zwei bis sieben Wochen.
Ein Großteil der Hepatitis A―Infektionen verlaufen ohne Beschwerden. Der Patient merkt also nicht, dass er sich mit dem Virus infiziert hat, und die Erkrankung heilt von selbst wieder aus.
Wenn sich Beschwerden entwickeln, treten zunächst unspezifische Erkrankungsanzeichen auf. Dazu zählen ein leichter Temperaturanstieg, Appetitverlust, Übelkeit, Erbrechen, Leistungsabfall und Druckschmerzen im rechten Oberbauch oder auch psychische Auffälligkeiten. Etwa eine Woche später verfärbt sich die Haut gelb (Ikterus, Gelbsucht), der Urin wird dunkel und der Stuhl hell (sog. Lehmstuhl). Die Gelbfärbung ist hierbei durch den Gallenfarbstoff bedingt, den die Leber nicht ordentlich verarbeiten kann.
Bei Beginn der Gelbfärbung der Haut fühlen sich die Patienten meist bereits besser. Diese Gelbsucht hält üblicherweise vier bis acht Wochen an. Die Hepatitis A verläuft in der Regel meist als milde Erkrankung. Chronische Verläufe wie bei anderen Arten der Hepatitis kommen nicht vor. Nur in sehr seltenen Fällen tritt akutes Leberversagen auf.
Eine spezifische Therapie bei dieser Viruserkrankung gibt es nicht. Jedoch ist eine regelmäßige Kontrolle der "Leberwerte" erforderlich.
Die Diätvorschriften (insbesondere Alkohol) sollten noch Monate weiter beachtet werden, um einen späteren Rückfall zu vermeiden.

### Hepatitis C

Ganz anders als bei Hepatitis A oder Hepatitis B ist die Situation bei einer anderen Variante der Gelbsucht, der Hepatitis C. Meist verläuft sie weniger gravierend als die Hepatitis B und sorgt eher für chronische denn für akute Beschwerden. Zuweilen kann aber auch sie zur Leberzirrhose oder gar zum Leberkrebs führen. Das Problem hierbei ist, dass es gegen Hepatitis C noch keinen Impfstoff gibt. Sie ist eine relativ neue Krankheit, denn es gibt sie erst seit etwa 30 Jahren. Manche Experten glauben, dass weltweit mittlerweile 400 Millionen Menschen mit dem Hepatitis C-Virus infiziert sind.
Viele HIV-Kranke sind auch mit Hepatitis B oder C infiziert. Der Verlauf von Hepatitis kann kompliziert werden durch die Leberbelastung infolge der HIV-Medikation. Dann kann die Hepatitis vorübergehend wieder aufflammen.

### Borreliose

Jedes Jahr erkranken etwa 40.000 Menschen in Deutschland an Borreliose. Eine halbe Million Menschen sind nach den Schätzungen von Selbsthilfeorganisationen chronisch betroffen.
Auslöser dieser heimtückischen Infektionskrankheit sind Bakterien namens Borrelium burgdorferi aus der Gruppe der Spirochaeten. Überträger des Bakteriums sind Zecken, die den Erreger während des Saugaktes nach einigen Stunden auf den Menschen übertragen. In Deutschland ist dies vor allem die Zecke Ixodes ricinus, auch gemeiner Holzbock genannt. Gegen diese Krankheit gibt es keinen zugelassenen Impfstoff und auch keine lebenslange Immunität. In den USA wurde ein entsprechender Impfstoff 2002 wegen angeblicher Impfkomplikationen wieder vom Markt genommen. Dieser Impfstoff war allerdings nur für den amerikanischen Erregerstamm geeignet und somit in Europa nicht anwendbar.
Die eigentliche Bezeichnung Lyme-Borreliose setzt sich dabei zusammen aus dem Namen des Ortes Lyme im US-Bundesstaat Connecticut, in dem in den 1970er Jahren diese Krankheit erstmals beschrieben wurde, sowie aus der Bezeichnung der Erkrankung als Borreliose, die auf die mikrobiologisch-systematische Einteilung des Erregers zurückgeht. Der Erreger wurde als "Borrelia burgdorferi" nach seinem Entdecker Willy Burgdorfer, einem Schweizer, benannt. Dieser hatte diese Bakterien 1981 in den USA entdeckt.
Die Lyme-Borreliose ist praktisch weltweit verbreitet. Es gibt inzwischen Berichte aus fast allen Teilen der Erde inklusive Australien, Südafrika und der Volksrepublik China. Klimatische Temperaturgrenzen scheinen für den Erreger nicht zu existieren. Die Durchseuchung der Zecken mit Borrelien weist ein Nord-Süd-Gefälle auf. Der höchste Durchseuchungsgrad in Deutschland wurde im süddeutschen Raum festgestellt. Hier liegt er zum Teil bei 30% bis 50%.
In nördlichen Regionen, wie zum Beispiel in Niedersachsen liegt die Durchseuchungsrate der Zecken bei etwa 10%. Mittlerweile sind mindestens vier humanpathogene Untergruppen des Erregers Borrelia burgdorferi bekannt, so genannte Genospezies.
Genaue Daten über die Erkrankungsrate und Ausbreitung des Erregers gibt es nicht, da flächendeckende, epidemiologische Daten fehlen. Nur in den Neuen Bundesländern und in Berlin besteht eine Meldepflicht für diese Erkrankung.
Ein großes Problem bei der genauen Feststellung der Borreliose ist die laborchemische (serologische) Unterscheidung zwischen einer abgeheilten Borreliose (Seronarbe) von einer noch aktiv therapiebedürftigen Borreliose. In der Serologie werden in der Routinediagnostik Antikörpertests eingesetzt. Das sind in der Regel der ELISA und der Westernblot. Solche Tests können nur die Antikörper messen, das heißt, feststellen, ob ein Erregerkontakt stattgefunden hat oder nicht. Es ist jedoch durch dieses Verfahren nicht möglich, den Krankheitsverlauf einer Borreliose zu kontrollieren. Deshalb ist es auch so schwierig, nach einer Behandlung mit Antibiotika festzustellen, ob diese wirksam waren und die Borreliose nun ausgeheilt ist. Hinzu kommt, dass die einzelnen Testverfahren nicht standardisiert sind und eine unterschiedliche Spezivität und Sensitivität aufweisen. Bei sehr sensitiven Tests besteht oftmals das Problem von so genannten Kreuzreaktionen. Das bedeutet, der Test zeigt ein positives Borrelien-Ergebnis an, der Betreffende hat aber noch keine Borreliose. Das wird durch andere Erreger, wie zum Beispiel durch andere Spirochaeten, wie Treponema pallidum oder Treponema denticola, Leptospiren, aber auch durch Eppstein-Barr-Virus oder Zytomegalivirus, verursacht. Genauso kommen falsche negative Ergebnisse vor. Die Serologie ist vor allem in den frühen Phasen nicht zuverlässiger als 30%. Neuere Tests haben inzwischen eine etwas höhere Zuverlässigkeit, die mit einer Sensitivität von 70% bis 80% angegeben wird. Dadurch werden gerade in der Frühphase viele Borreliose-Fälle übersehen. In späteren Stadien ist die Sensitivität in der Regel höher. Bei einem Verdacht auf eine Neuroborreliose ist in der Regel eine Nervenwasseruntersuchung angezeigt. Allerdings kann es heirbei bei ca. 30% zu falschen Ergebnissen kommen. Von einigen Labors wird neuerdings zur Feststellung der Erregeraktivität der so genannte LTT (Lymphozythentransformationstest) angeboten, dessen Zuverlässigkeit jedoch bisher noch nicht ausreichend durch Studien nachgewiesen werden konnte. Über das Internet werden zunehmend obskure Borrelien-Testverfahren vermarktet, wie zum Beispiel der VCS-Test oder Testverfahren mittels Elektroakupunktur, von denen dringend abzuraten ist.
Bei dem Krankheitsverlauf der Borreliose können drei Stadien unterschieden werden.

Das erste Stadium ist durch eine Lokalinfektion gekennzeichnet.
Nach der Übertragung des Erregers kann es zu einer lokalen Infektion der Haut kommen, die mit einem charakteristischen Hautausschlag, dem so genannten Erythema migrans (Wanderröte) einhergeht. Das Erythem verschwindet manchmal ohne Therapie, kann aber auch über Monate bestehen. Es dehnt sich meist langsam um die Stichstelle von einem Zeckenstich aus (daher der Name). Das "Erythema migrans" ist ein eindeutiges Symptom für eine Borrelieninfektion. Allerdings gibt es viele Infektionen mit Borrelien ohne dieses eindeutige Zeichen einer Infektion. Im ersten Stadium kann die Borreliose mit Antibiotika behandelt werden. Notwendig ist jedoch eine ausreichend lange und hoch genug dosierte Therapie.

Im zweiten Stadium wird der Erreger im Körper verstreut.
Nach einer Zeit von bis zu zehn Wochen können sich die Erreger im ganzen Körper ausbreiten. Der Patient leidet an grippeähnlichen Symptomen wie Fieber und Kopfschmerzen, was die Erkennung der Krankheit erschwert. Charakteristisch sind starke Schweißausbrüche. Durch die Ausbreitung im Körper kann es zu einem Befall der Organe, der Gelenke und Muskeln sowie des zentralen und peripheren Nervensystems kommen. Leitsymptome in diesem Stadium sind oftmals das so genannte Bannwarthsyndrom mir starken, radikulitischen Schmerzen und die Facialisparese, die sich in einem schiefen Gesicht zeigt. Typisch sind auch von Gelenk zu Gelenk springende Arthritiden und Myalgien. Weiterhin kommt es oft zu Störungen des Tastsinns, Sehstörungen und Herzproblemen, wie Sinustachykardien und Karditis, was sich manchmal durch Herzklopfen und hohen Blutdruck sowie Pulsbeschleunigung bemerkbar macht. Das körpereigene Immunsystem ist oftmals in diesem Stadium allein nicht mehr in der Lage, die Infektion zu bewältigen. Borrelien scheinen sich nur kurz im Blut aufzuhalten und sich sehr schnell im Bindegewebe festzusetzen. Problematisch ist hier auch die so genannte Neuroborreliose, die zu vielfältigen Erkrankungen des peripheren und zentralen Nervensystems führen kann. Deshalb wird in diesem Stadium ausreichend mit Antibiotika behandelt. Die Wahl des Antibiotikums richtet sich nach dem Befall und der Erkrankungsform.

Das dritte Stadium ist gekennzeichnet durch eine chronische Infektion.
Wenn die Borreliose nicht rechtzeitig erkannt und behandelt wird, kann es zu einer Erregerpersistenz kommen. Das heißt, die Krankheit kommt immer wieder. Das kann sich über Monate und Jahre hinziehen und verursacht dem Patienten starke Schmerzen durch Muskel-und Gelenkentzündungen. Außerdem kommt es häufig zu einem Befall des zentralen und peripheren Nervensystems. Hier können Erkrankungen auftreten wie Polyneuropathie, Meningitis oder Enzephalitis. Ebenso sind chronische Erkrankungen der Sinnesorgane und der Gelenke und Muskeln möglich. Die chronischen Erkrankungen der Gelenke werden Lyme-Arthritis genannt. Es kann aber auch zu einer entzündlichen Bursitis oder Arthrose kommen.

Wenn im zweiten Stadium nicht rechtzeitig eine Heilung zustande kommt, so können bleibende Organschäden entstehen, die dann trotz Therapie nicht geheilt werden können.

### Multiple Sklerose

Vermutlich müssen mehrere Bedingungen und Einflüsse zusammentreffen, damit die Multiple Sklerose (MS) ausgelöst wird. Das Durchschnittsalter bei Beginn der Erkrankung liegt im frühen Erwachsenenalter. Frauen erkranken daran öfter als Männer, wobei sich hierfür noch keine Erklärung fand. Die MS ist die häufigste, entzündliche, neurologische Erkrankung in Deutschland.
Es gibt Untersuchungen, die aufzeigen, dass in bestimmten Breitengraden der Erde MS häufiger auftritt, in anderen dagegen selten. So ist die MS in Mitteleuropa, Nordeuropa und Nordamerika häufiger als in Südeuropa, Südamerika und Afrika.

Dabei sollte man aber nicht übersehen, dass es in den südlichen Ländern schlechtere Diagnosemöglichkeiten gibt. Eine passende Theorie dazu ist, dass das Ausmaß der Sonneneinstrahlung einen Einfluss auf die Entwicklung von MS hat. Zur Erkrankung gibt es wegen der vielen unterschiedlichen Verläufe von MS auch verschiedene Ursachen-Theorien. Zu nennen sind hier die Autoimmuntheorie, die Virus-Hypothese, die genetischen Faktoren, die Stoffwechseltheorie und die psychosomatischen Theorien.
Die Forschung von Wissenschaftlern und Ärzten kommt nur mühsam voran, zum einen, weil man das zentrale Nervensystem am lebenden Menschen nicht operativ öffnen kann und zum zweiten, weil die notwendigen finanziellen Mittel fehlen.
Des Weiteren wird kolportiert, dass Innovationen auf diesem Gebiet die Interessenlage großer Konzerne tangieren.

Die Multiple Sklerose kann in vielen Erscheinungsformen auftreten.
Bei ca. 40% aller MS-Erkrankungen kommt es zur Entzündung eines Sehnervs, Bemerkbar macht sich die Sehnerventzündung durch einen Verlust an Sehschärfe und das Sehen verschwommener Bilder. Es können Schmerzen bei Augenbewegungen auftreten. Es kann auch zu Lähmungen der Augenmuskeln kommen. Dabei sehen die Betroffenen meist Doppelbilder. Augenzittern kann auftreten, das jedoch nur bei starker Ausprägung zu Sehstörungen führt. Diese Beschwerden können völlig verschwinden, aber auch immer wieder auftreten. In manchen Fällen besteht ein Fremdkörpergefühl im Auge.
Im Hirnstamm, der Verbindung zwischen Gehirn und Rückenmark, verlaufen unter anderem Nervenfasern für die Berührungsempfindlichkeit des Gesichts. Werden diese von MS befallen, kann es zu heftigen Schmerzattacken im Gesicht kommen, besonders bei Berührung. Hier reicht schon der Kontakt der Haut mit längeren Haaren.
Schwindel und Brechreiz können vorkommen, wenn die Nervenbahnen geschädigt werden, die eine Verbindung zum Gleichgewichtsorgan herstellen. Entzündungsherde im Hirnstamm können weiterhin zu einer verwaschenen Sprache führen. Es kann auch zu Sprachstörungen kommen, die nur für kurze Zeit, oft mehrmals am Tag, auftreten.

Werden Nervenfasern im Bereich des Kleinhirns oder in seinen Verbindungsbahnen von der MS befallen, treten Koordinationsstörungen auf. Sichtbar ist dies zum Beispiel durch Zittern, durch einen unsicheren Gang und durch eine abgehackte oder lallende Sprache. Je nach Ausprägungsgrad sind Sprachstörungen besonders behindernd und können den Erkrankten sozial isolieren. Der Erkrankte wird damit des wichtigsten Kommunikationsmittels mit seiner menschlichen Umwelt beraubt. Es erfordert hierbei sowohl vom MS-Kranken als auch vom Gesprächspartner viel Geduld, immer wieder zu versuchen, das Gespräch langsam und ruhig zu führen.

Es bestehen Schwierigkeiten, gezielte Bewegungen auszuführen, wie zum Beispiel Getränke an den Mund zu führen. Das Zittern kann so stark sein, dass geplante Bewegungen nicht ausgeführt werden können. Das führt dann oft zu Wutausbrüchen, wobei der Betroffene sich selbst die Schuld an dem Unvermögen gibt. Oft wird dieses Unvermögen durch erneute Versuche verstärkt, weil es einfach nicht akzeptiert werden kann, dass der Körper bestimmt, was vollführt werden kann und was nicht. Es können Unsicherheiten beim Sitzen, Stehen oder Gehen auftreten. Der unsichere Gang bei einem an MS Erkrankten wird von Außenstehenden oft mit dem Gang eines Betrunkenen verwechselt. Dies führt zwangsläufig zu peinlichen und kränkenden Situationen. Viele MS-Betroffene fühlen sich beim Gehen behindert, gerade dann wenn sie sich besonders darauf konzentrieren.

Bei Befall des Rückenmarks mit Multipler Sklerose können grundsätzlich zwei verschiedene Störungen auftreten. Auf der einen Seite kann es zu Störungen der Empfindungswahrnehmungen kommen, auf der anderen Seite zu Störungen der Bewegung der Muskeln.
Bei einer Entzündung des Rückenmarks kann es zu Sensibilitätsstörungen kommen in der Form von Kribbeln und Taubheitsgefühlen in der Muskulatur. Diese können dann als Schwere, als Kälte, Beengung oder Spannung empfunden werden. In den Händen kann es zu einer Empfindung kommen, als sei in und zwischen den Fingern Pelz, oder als seien die Hände mit einem Pelz überzogen. Es kann auch zu einer schmerzhaften Missempfindung kommen, die meist als Brennen in Erscheinung tritt. Solche Missempfindungen sind besonders störend, da bei jeder Berührung mit den Händen ein unangenehmes Gefühl entsteht.
Nervenstörungen der Muskeln führen zu unterschiedlichen Beschwerden. Die Muskeln können schwach und schlaff werden. So können die Beine wegknicken oder ein Fuß hängt schlaff nach unten. Aber auch eine beständige Anspannung (Spastik) ist möglich, so dass bestimmte Bewegungen nicht mehr oder nur noch unter Anstrengung machbar sind. Dazu gehört u.a. die Streckspastik oder Beugespastik im Bein. In diesem Fall bekommt man das Bein nicht mehr gestreckt oder gebeugt wenn man aufstehen will.
Da die motorischen Störungen meist das Gehvermögen beeinflussen, beeinträchtigen sie oft auch die allgemeine Leistungsfähigkeit.
Weitere Beschwerden bei Befall des Rückenmarks von MS sind Blasenstörungen und Darmstörungen. Die Angst vieler MS-Kranker, bei einem Harndrang nicht rechtzeitig eine Toilette besuchen zu können, führt oft dazu, dass sie nicht mehr aus dem Haus gehen möchten.
Harnträufeln und Harndrang oder der vollständige Verlust über die Kontrolle der Harnblase oder des Darms sind die Beschwerden, die zu den größten sozialen Beeinträchtigungen führen können. Auch der Harn-oder Blasenverhalt ist unangenehm. Besonders der Harnverhalt, bei dem die Blase nicht vollständig entleert werden kann, kann zu schmerzhaften Blaseninfektionen führen.

Es ist deshalb die Aufgabe der vorliegenden Erfindung, Mittel und Verfahren zur Behandlung von Hepatitis C, Borreliose und Multipler Sklerose auf der Basis biogener Grundstoffe zur Verfügung zu stellen, die es erlauben, größere Mengen an Wirkstoff zu produzieren.

Diese Aufgabe wird von dem Wirkstoffen gemäß Anspruch 1 sowie dem Verfahren gemäß den Ansprüchen 16-19 gelöst.

Es ist auch eine Aufgabe der Erfindung, verbesserte Mittel und Verfahren zur Bekämpfung der oben genannten Krankheiten, insbesondere von Borreliose, auf der Basis biogener Gifte bereitzustellen.

Diese Aufgabe wird von den Wirkstoffen gemäß den Ansprüchen 2 bis 15 sowie dem Verfahren gemäß den Ansprüchen 20-24 gelöst.

Ein erster Aspekt der vorliegenden Erfindung betrifft einen pharmazeutischen Wirkstoff zur Behandlung von Hepatitis A, Hepatitis C, Borreliose und/oder Multipler Sklerose, enthaltend in einer pharmazeutisch wirksamen Menge zumindest ungereinigtes Drüsensekret, gewonnen von Tieren der Klasse Diplopoda.

Nach den Insekten und den Spinnentieren sind die Myriapoda, zu denen auch die Diplopoda (Tausendfüßer) und Chilopoda (Hundertfüßer, Scolopender) zählen, die drittgrößte Tiergruppe mit geschätzten 80.000 Arten.
Tausendfüßer gibt es schon seit rund 410 Millionen Jahren (Erdzeitalter Silur). Diese waren eine der ersten Landbewohner und stellen somit eine sehr urtümliche Tiergruppe dar. Arthropleura lebte vor 310 Millionen Jahren und erreichte eine Länge von 2 Metern bei einer breite von 50 cm. Es war der größte bislang bekannte Landarthropode, der jemals auf dieser Erde lebte. Er war ein Räuber, der in der Lage war, Beute in der Größe eines Rehs zu erlegen.
Der größte bekannte zur Zeit lebende Tausendfüßer ist Archispirostreptus gigas mit 30 cm Länge. Die kleinsten Vertreter erreichen gerade die Länge von 1 mm. Diplopoda heißt übersetzt Doppelfüßer. Bei der Anpassung an ihren Hauptlebensraum, das Bodenreich, sind jeweils 2 Segmente mit einander verschmolzen, so genannte Diplosegmente, welche jeweils 2 Beinpaare tragen und dem Körper beim Gehen mehr Stabilität geben.
Sie heißen zwar Tausendfüßer, aber keine Art hat 1000 Beine. Lediglich Illacme plenipes (Siphonophorida; Californien) trägt an 192 Körperringen beachtliche 750 Beine, womit sie dem Namen Tausendfüßer annähernd gerecht wird.
Diplopoden findet man auf allen Kontinenten bis auf den Nord- und Südpol.
Sie bewohnen die unterschiedlichsten Lebensräume und ernähren sich überwiegend von abgestorbenem pflanzlichem und auch tierischem Material. Es gibt aber auch einige Nahrungsspezialisten, die sich auf Kot (zum Beispiel Guano) spezialisiert haben oder sich von Pilzen und Flechten ernähren. Tausendfüßer sind meist sehr träge, lichtscheu und dämmerungs- und nachtaktiv. Da Tausendfüßer nicht gut gegen Transpiration (Wasserverlust) geschützt sind, sind sie an relativ feuchte Standorte gebunden. Wasser stellt also für ihre Lebensweise den limitierenden Faktor dar.
Tausendfüßer spielen eine wichtige Rolle für unsere Böden. Sie tragen zur besseren Durchlüftung der Böden bei, tragen Mineralboden in die oberen Bodenschichten und zersetzen und mischen organisches mit anorganischem Material in ihrem Darm. In manchen Gebieten dieser Erde sind Tausendfüßer die Hauptzersetzer der Laubschichten. In Mitteleuropa zersetzen sie dagegen nur 1 % bis 5% der jährlich anfallenden Laubstreu. Da Tausendfüßer mehr oder weniger in ihrer Nahrung leben und sie nur in begrenzten Gebieten und Landschaften vorkommen, dienen sie oft als zoogeographische Indikatoren.
Zu den Fressfeinden der Tausendfüßer gehören mehrere Vogelarten, Skorpione und
verschiedene Reptilien, wie Schildkröten, Leguane und Kaimane; außerdem noch die Haselmaus, einige Wanzenarten, welche gruppenweise einen Tausendfüßer anfallen, anstechen und aussaugen und einige Ameisenarten.
Zur Abwehr von Feinden sondern Tausendfüßer Abwehrsekret, oder auch Wehrsekret genannt, ab. Aus den seitlichen Öffnungen der Körperringe kann eine, je nach Art unterschiedliche, Menge an Wehrsekret abgegeben oder teilweise auch einige cm weit gespritzt werden. Das meist gelbliche Sekret enthält Benzochinone und Chinone die einen üblen Geruch haben und auf der Haut gelbbraune bis violettfarbene Flecken hinterlassen, die nach einigen Tagen bis Wochen wieder verschwinden. Das Sekret ist auch schleimhautreizend und sollte daher nicht in die Nase, die Augen oder den Mund gelangen.
Tausendfüßer sondern ständig in gasförmiger Form Wehrsekret ab, um sich vor Pilzen und Bakterien zu schützen.
Da Tausendfüßer ein starres Exoskelett besitzen, welches nicht mitwächst, müssen sie sich häuten, um zu wachsen. Es dauert dann einige Stunden bis Tage, bis das neue Exoskelett ausgehärtet ist und die Tiere wieder auf der Oberfläche erscheinen. Insgesamt können die Tiere mehrere Wochen für die Häutung benötigen, bis das neue Exoskelett wieder komplett ausgehärtet ist.
Es gibt sowohl kurzlebige als auch langlebige Arten. Viele heimische Arten als auch die tropischen Arten erreichen oft ein Alter von 4 bis 8 Jahren. Archispirostreptus gigas ist wohl der am ältesten werdende Tausendfüßer mit geschätzten 10 Jahren.

Im Prinzip sind die Hundertfüßer mit den Tausendfüßern nicht so nahe verwandt wie man vom Namen her glaubt.
Es gibt wesentliche Unterschiede zwischen diesen beiden Familien. So haben Hundertfüßer nur ein Beinpaar pro Segment, wohingegen Tausendfüßer zwei Beinpaare an einem Diplosegment haben.

Hundertfüßer haben eine weiche Cuticula ohne Kalkeingerungen, im Gegensatz zu den Tausendfüßern.
Hundertfüßer ernähren sich ausschließlich räuberisch und die Fangklauen sind aus dem ersten Laufbeinpaar im Laufe der Zeit gebildet worden.
Die Hundertfüßer tragen ihre Geschlechtsorgane im letzten Segment, die Tausendfüßer hingegen im dritten. Außerdem sind die Antennen der Hundertfüßer um ein Vielfaches länger als die der Diplopoden.

Da sich die gesamte Klasse Diplopoda zur Herstellung des erfindungsgemäßen Wirkstoffs eignet, wird im Folgenden die grundlegende Systematik ihres Stammbaums nach Enghoff dargelegt:
Klasse: Diplopoda
   Unterklasse: Penicillata
      Ordnung: Polyxenida
   Unterklasse: Chilognatha Pentanzonia
      Ordnung: Giomeridesmida
      Ordnung: Giomenda
      Ordnung: Spaerotheriida Helminthomorpha Colobognatha
      Ordnung: Platydesmida
      Ordnung: Polyzoniida
      Ordnung: Siphonophorida Eugnatha
      Überordnung: Nematophora
      Ordnung: Stemmiulida
      Ordnung: Callipodida
      Ordnung: Choreumatida
      Überordnung: Merocheta
      Ordnung: Poldesmida
      Überordnung: Juliformia
      Ordnung: Julida
      Ordnung: Spirobolida
      Ordnung: irostrepida

Die oben genannten Arten, Familien und Gattung sind bevorzugt.

Der Grundstoff für die Gewinnung des erfindungsgemäßen Wirkstoffs wird bei den angeführten Diplopoden auf die folgende Weise gewonnen:

Es ist zuerst darauf zu achten, dass das betreffende Tier nicht verschmutzt ist. Dann ist das Tier am Rücken zu nehmen und umzudrehen und unter Reinraumbedingungen das Drüsensekret an den Beinen abzunehmen. Bei kleinen Tieren ist hierbei ein steriles Wattestäbchen geeignet, bei großen Tieren ist die Verwendung einer Pipette passender. Die Temperatur sollte dabei im Bereich von 25 bis 28°C liegen. Liegt die Temperatur über 30°C, können sich einzelne Bestandteile des Sekrets verflüchtigen. Liegt die Temperatur dagegen unter etwa 20°C werden einige wichtige Bestandteile des Sekrets von dem Tier nicht abgegeben und/oder synthetisiert.
Auf diese Weise lassen sich von einer Person etwa 500 Tiere am Tag melken.
Das so erhaltene Sekret wird nicht gereinigt und nicht erhitzt, sondern so gefriergetrocknet, wie es gewonnen wird.
1 mg des so gewonnen Drüsensekrets wird unter Rühren mit 500 mL 0,9%iger NaCl-Lösung vermengt und ergibt die gebrauchsfertige Lösung. Diese ist etwa 9 Monate haltbar.
Für eine Behandlung reicht eine Dosis von 2 mL.

Zur Behandlung von Borreliose kann die Wirkung des Diplopodensekrets noch weiter verbessert werden

Der erfindungsgemäße Wirkstoff setzt sich hierbei aus zwei Bestandteilen zusammen.

Der erste Bestandteil wird, wie bereits oben beschrieben, aus Diplopoden gewonnen. Besonders geeignet sind hierfür die Sekrete afrikanischer Tausendfüßer-Arten.

Zu dem Sekret der Diplopoden wird ein zweiter Bestandteil gemischt. Dieses wird im Wesentlichen setzt sich dieser durch einen pharmazeutischen Wirkstoff zusammen, enthaltend zumindest ein Peptidtoxin und/oder zumindest eine hierzu antagonistisch oder synergistisch wirkende Substanz und/oder eine Durchdringungssubstanz in einer pharmazeutisch wirksamen Menge, wobei zumindest ein Peptidtoxin und/oder zumindest eine antagonistisch oder synergistisch wirkende Substanz aus dem Gift der folgenden Tiergattungen stammen:
a) unter den Spinnen der Familie Loxoscelidae besonders die Gattung Loxosceles, insbesondere, Loxosceles rufipes , Loxosceles sp. (Mallorca), Loxosceles sp. (Menorca), Loxosceles sp. (Ibica)
b) unter den Scorpionen die Gattung Euscorpius, insbesondere mit den Arten Euscorpius germanus, Euscorpius spp. Mallorca I und Mallorca II, Euscorpius balearicus, sowie Euscorpius spp. (Mallorca, Menorca, Ibica)
c) unter den Skolopendern die Gattung Scolopendra insbesondere mit der Artengruppe um die Art cingulata,
d) unter den Spinnen der Familie Scytodiae und unter der Familie Lycosidae insbesondere die Arten der Gattung Lycosa und
e) unter den Spinnen die Arten der Gattung Lycosa spp. (Mallorca, Menorca)
f) unter den Spinnen der Familie Dysderidae insbesondere die Arten der Art Dysdera spp. (Mallorca, Menorca, Ibica).

Dies hat den Vorteil, dass dadurch das von der Natur gegebene Zusammenspiel von Peptidtoxinen und dazu antagonistisch oder synergistisch wirkenden Substanzen eines einzigen Organismus ausgenutzt werden kann.

Gemäß der vorliegenden Erfindung können das Peptidtoxin oder die hierzu antagonistisch bzw. synergistisch wirkende Substanz und/oder die Durchdringungssubstanz aber auch aus einem anderen Organismus stammen oder synthetisch oder gentechnisch hergestellt worden sein. Beispielsweise kann das Peptidtoxin das Schlangengift Captopril sein oder die antagonistisch wirkende Substanz kann eine Hyaluronidase aus Kobragiften oder synergistisch wirkendes Loxosceles (Mallorca)-Toxin sein.

Die antagonistisch oder synergistisch wirkende Substanz und/oder die Durchdringungssubstanz ist bevorzugt eine Phospholipase oder eine Hyaluronidase oder eine Kombination beider Substanzen. Unter einer Durchdringungssubstanz wird eine Substanz verstanden, die den Wirkstoffen helfen können, besser durch das Gewebe und die Zellwände und Zellmembranen zu gelangen. Bevorzugt sind hier Durchdringungsenzyme wie Phospholipasen und/oder Hyaluronidasen. Die Durchdringungssubstanz kann auch synthetischen Ursprungs sein. Besonders bevorzugt sind die Phospholipasen der Loxoscelesarten der Balearen.
Weiterhin ist bevorzugt, dass die antagonistisch bzw. synergistisch wirkende Substanz und/oder die Durchdringungssubstanz eine Mischung aus den im Gift von Spinnen, Skorpionen, Tausendfüsslern und Skolopendern der in dieser Erfindung genannten Arten, vorhandenen Phospholipasen und Hyaluronidasen und/oder Toxinen ist.
Bevorzugt werden das Peptidtoxin und die hierzu antagonistisch oder synergistisch wirksame Substanz und/oder die Durchdringungssubstanz durch ein Fraktionierungsverfahren aus den Spinnen-, Skorpion-, Tausendfüssler- und Skolopendergiften erhalten, und es ist weiterhin bevorzugt, dass der pharmazeutische Wirkstoff ein Peptidtoxin und eine hierzu antagonistisch oder synergistisch wirkende Substanz enthält, die aus verschiedenen Fraktionen stammen. Dadurch kann der pharmazeutische Wirkstoff in seiner Wirkung vorteilhafterweise auf die zu behandelnde Tumorart und/oder Tumorgröße abgestimmt werden.
Das Peptidtoxin und die hierzu antagonistisch oder synergistisch wirkende Substanz können durch an sich bekannte Fraktionierungsverfahren zur Auftrennung von Proteinen aus dem Spinnen-, Skorpion-, Tausendfüssler- und Skolopendergift-Rohgemisch (Giftcocktail) erhalten werden. Bevorzugt ist, dass das Peptidtoxin und die hierzu antagonistisch oder synergistisch wirkende Substanz durch Gelchromatographie, HPLC, Affinitätschromatographie und/oder lonenaustauschchromatographie erhalten werden.

Es ist weiter bevorzugt, dass der pharmazeutische Wirkstoff übliche Träger- und Hilfsstoffe enthält. Bevorzugt ist, dass der pharmazeutische Wirkstoff weitere Wirkstoffe wie Antibiotika, Antimykotika, Antituberkulotika, Mittel gegen Parasiten, Zytostatika, Aminosäuren, die Wundheilung begünstigende Enzyme und/oder Mitosehemmstoffe enthält .Bevorzugt sind dabei Penicillin/Streptomycin, Poymyxin/Gentamycin, Glutamin (5%), Mitopodozid, Vinca rosea-Alkaloide, Bromelainar oder Bromelains.
In dem erfindungsgemäßen pharmazeutischen Wirkstoff werden das Peptidtoxin und die antagonistisch oder synergistisch wirkende Substanz und/oder die Durchdringungssubstanz in Kombination miteinander eingesetzt. Es ist aber auch möglich, die Einzelsubstanzen in pharmazeutischen Wirkstoffen zu benutzen und sich hierbei die speziellen Wirkungen der Substanzen für eine therapeutische Anwendung nutzbar zu machen.

Es ist auch möglich die beschriebenen Wirkstoffe chemisch-synthetisch oder durch gentechnologische Methoden in rekombinierter Form herzustellen. Wie bei chemischen Substanzen üblich, umfasst die vorliegende Erfindung auch Derivate und Salze der erfindungsgemäß bereitgestellten Substanzen. Beispielsweise kann das Peptidtoxin ein oder mehrere Additionen, Substitutionen und/oder Deletionen von Aminosäuren umfassen, wobei sichergestellt sein muss, dass die erfindungsgemäße medizinische Wirkung erhalten bleibt.
Die Gewinnung des Peptidtoxins und der hierzu antagonistisch oder synergistisch wirkenden Substanz(en) erfolgt durch in der chemischen Verfahrenstechnik übliche Methoden. Hierzu gehören insbesondere Fraktionierungsverfahren; es sind aber auch andere Verfahren einsetzbar, beispielsweise immunologische Verfahren, um die gewünschten Substanzen aus dem Gesamtgift-Cocktail herauszuholen.

Ein bevorzugtes Verfahren zur Herstellung eines erfindungsgemäßen Wirkstoffes, enthaltend zumindest ein Peptidtoxin und/oder zumindest eine hierzu antagonistisch oder synergistisch wirkende Substanz und/oder eine Durchdringungssubstanz, wobei zumindest ein Peptidtoxin und/oder zumindest eine antagonistisch oder synergistisch wirkende Substanz und/oder eine Durchdringungssubstanz aus dem Gift von Tieren der oben unter a) bis f) genannten Familien und Gattungen stammen, weist folgende Schritte auf:
Gewinnen eines Gift-Rohgemischs durch an sich bekannte Verfahren sowie Fraktionierung der Mischung, um das Peptidtoxin und die hierzu antagonistisch oder synergistisch wirkende Substanz in möglichst voneinander getrennten Fraktionen zu erhalten; wahlweise
Kombination verschiedener Fraktionen miteinander oder mit aus anderen Organismen stammenden Peptidtoxinen oder antagonistisch oder synergistisch wirkenden Substanzen, um einen pharmazeutisch wirksamen Wirkstoff zu erhalten.

Die betreffenden Giftarten enthalten verschiedene Peptidtoxine und verschiedene hierzu antagonistisch oder synergistisch wirkende Substanzen und andere, ebenfalls medizinisch-therapeutisch relevante Wirkstoffe. Alle diese Substanzen können in einem bestimmten, vom Fachmann zu bestimmenden Verhältnis in einem medizinischen Wirkstoff therapeutisch eingesetzt werden.
Es wird darauf hingewiesen, dass das Fraktionierverfahren lediglich beispielhaft eine Möglichkeit zur Gewinnung der Peptidtoxine und der hierzu antagonistisch oder synergistisch wirkenden Substanzen aufzeigt. Weitere Ausgestaltungen sind möglich.

Dabei ist bevorzugt, dass das verwendete Gift-Rohgemisch aus weiblichen Tieren der unter a) bis f) genannten Familien und Gattungen gewonnen wird. Dies ist vorteilhaft, da weibliche Exemplare der verwendeten Arten mehr Gift produzieren als männliche Exemplare.

### Beispiel 1: Gewinnung des Sekrets von Diplopoden

Das Wehrsekret des betreffenden Tausendfüßers, insbesondere des afrikanischen Tausendfüßlers, wird gewonnen indem man das Tier umdreht und das Sekret, das über die Chitindrüsen ausgeschieden wird, mittels eines Wattestäbchens zwischen den Beinen abtupft.
Die Tiere dürfen im Terrarium nicht zu eng gehalten werden. Die Humus-Sand-Mischung des Bodens muss mindestens 15 cm hoch sein. Über dem Boden ist teilweise Laufstreu aufzulegen. Wurzeln und Äste vervollständigen die Einrichtung. Der Fütterung dienen Bio-Obst und Gemüse. Eventuell kommt ein spezieller Futterbrei zum Einsatz (ABiTec).

### Beispiel 2: Gewinnung des Gift-Rohgemisches

Zur manuellen Melkung wurden subadulte Weibchen der unter a) bis f) genannten Familien und Gattungen mit den Fingern einer Hand in Rückenlage fixiert und durch Berühren mit der stumpfen Seite einer auf eine sterile Spritze aufgesetzten sterilen Kanüle an den Chelizeren zur Abgabe des Giftes stimuliert. Die Raumtemperatur betrug meist etwa 21 bis 27 Grad, die Luftfeuchtigkeit 50% bis 70%. Die Tageszeit spielte keine Rolle.

Dabei war es bevorzugt, dass die Stimulationszeit nicht länger als 90 Sekunden dauerte, da sonst das betreffende Tier einem unnötigen Stress ausgesetzt würde. Nach Erscheinen des Gifttropfens an den Giftklauen wurde dieser mit der Spritze über die Kanüle aufgezogen. Für jedes Tier wurde eine neue Spritze mit neuer Kanüle verwendet. Anschließend wurde die Kanüle mit der Kanülenschutzhülle wieder verschlossen. Die verschlossene Spritze samt aufgezogenem Gift wurde direkt anschließend in einen Exsikator verbracht. Dieser wurde dann für mindestens 12 Stunden in einer Tiefkühltruhe bei mindestens 14 Grad Celsius aufbewahrt.
Die Skolopender und Skorpione wurden gemolken, wie es in der deutschen Offenlegungsschrift DE 10 2005 011 111.4 beschrieben ist. Bei der Spritze mit dem gefrorenen Gesamtgift wurde nach Entnahme aus der Tiefkühltruhe die Kanülenschutzhülle entfernt. Die Kanüle wurde in Lösungsmittel, z.B. Proteinlösungsmittel (Lösungsmittel für Protein-Säulenchromatographie: 0,25 M Tris/HCL, pH 6,5 bis 7,3, 1,92 M Glycin , in destilliertem, deionisiertem Wasser) eingetaucht und 1 ml aufgezogen. Wegen Denaturierung wird kein SDS im Puffer verwendet. Dadurch wurde Gift in Lösung erhalten. Im Anschluss wurde die Kanüle entfernt. Die so aufbereiteten einzelnen Giftlösungen in Spritzen (5 Stück) wurden durch Ausdrücken (Ausspritzen) in einem sterilen, sauberen Teflonvial bei Raumtemperatur gesammelt. Das verschlossene Teflonvial wurde anschließend auf einem Vortex ohne Schaumbildung 30 Sekunden lang geschüttelt, wobei eine homogene Lösung erhalten wurde.
Nach der Durchmischung wurde die gesamte Lösung über einen Plexiglastrichter (um Kontamination zu vermeiden) in eine stehende, transparente Plexiglassäule , die einen Innendurchmesser von 1,5 cm, eine Wandstärke von 2 mm und eine Höhe von 50 cm aufwies und unten konisch bis auf 1,5 mm zulaufend, offen war, gefüllt mit 20 mL Gel eingebracht. (Einzelheiten: Aca 34; Matrix 3% Acrylamid 4% Agarose; Fraktionierungsbereich (MW): Proteine : 20 bis 350 kDa; Ausschlussgrenze: 750 kDa; Kügelchendurchmesser: 60-140 Mikrometer). Die so eingebrachte Giftlösung durchlief das Gel und verdrängte dabei den im Gel befindlichen Puffer. Die Säule kann dabei im Durchmesser und der Höhe leicht variieren.

Nach vollständigem Eindringen der Giftlösung in das Gel wurden zusätzliche 165 mL Lösungsmittel (0,25M Tris/HCL, pH 6,5 bis 7,3, 1,92 M Glycin) auf die Säule gebracht. Dieses zusätzliche Lösungsmittel verdrängt bei seinem Durchlauf durch das Gel die darin befindliche Giftlösung. Die ersten 15 ml, die unten aus der Säule liefen, waren Restpuffer und wurden verworfen. Nach diesen 15 ml wurden 40 Fraktionen zu je 4 ml aufgefangen. Die Trennung in jeweils 4 ml war bedingt durch die physikalischen und chemischen Eigenschaften der einzelnen Fraktionen, nachgewiesen durch Elektrophorese, bevorzugt SDS-PAGE. Als Auftragspuffer zum Peptidbindungs- und Proteinschutz wurde Roti Load 1 + 2 (Carl Roth GmbH & Co KG, Karlsruhe: SDS-, Glycerol-, Bromphenolblau, Phosphatpuffer, Roti Load 1 mit Mercaptoethanol, Roti Load 2 ohne Mercaptoethanol) verwendet. Die einzelnen Fraktionen wurden in steril sauberen, verschraubbaren 5 ml Teflonvials getrennt aufgefangen. Die Qualitätskontrolle der Einzelfraktionen erfolgte mittels Elektrophorese.

Um die Struktur der Substanzen aufzuklären, wurden die einzelnen Fraktionen über eine HPLC-MS-MS sowie über eine DAD-UV/-Spektrometrie (DAD bzw.DADI: direct Analysis of Daughter Ions, direkte Analyse von Tochterionen) untersucht. Bekannte Substanzen konnten in höherem Molekulargewichtsbereich (ab 10.000 Da) nicht dargestellt werden. Allerdings deuten die Grundgerüstbestimmungen auf die Zugehörigkeit der Substanzen zum Polypeptidtyp mit toxischen Komponenten (= Polypeptidtoxinen) und andererseits zum Phospholipase- und Hyaluronidase-Typ hin.
Fraktionen mit gleicher Zusammensetzung können zusammen gesammelt werden. Für die weitere Verarbeitung und Lagerung wurden die einzelnen Fraktionen gefriergetrocknet.
Die zu lyophilisierende Fraktion wurde in einem offenen, mit perforierter Aluminiumfolie locker bedeckten, Teflonvial auf minus 22 Grad Celsius gekühlt. Zur sicheren Durchfrierung der Probe wurde eine Kühlzeit von 11 Stunden eingehalten. Dann wurde ein Vakuum von 0,200 mbar angelegt. Nach Erreichen des Vakuums wurde die Fraktion auf plus 4 Grad Celsius erwärmt und mindestens 24 Stunden unter Aufrechterhaltung des Vakuums auf dieser Temperatur gehalten. Nach dem Gefriertrocknungsvorgang wurde das Teflonvial mit der lyophilisierten Fraktion luftdicht verschraubt. Die Lagerzeit beträgt bei Raumtemperatur ca. 6 Monate, bei plus 7 Grad Celsius ca. 1 Jahr und bei minus 14 Grad Celsius ca. 15 Jahre.

### Beispiel 3: Herstellung des erfindungsgemäßen Wirkstoffes

Die Herstellung des erfindungsgemäßen Wirkstoffes erfolgt in den folgenden Schritten:
1) Das Material des Bestandteils aus Beispiel 1, das aus den Chitindrüsen von Tausendfüßern gewonnen wurde, wird in einer Kochsalzlösung mit 10% des Bestandteils aus Beispiel 2 bis zu Sättigung gelöst.
2) Diese Lösung wird dann auf 50°C für mindestens 3 Tage in einem sterilen Abzug vorsichtig erwärmt
3) Anschließend wird diese Lösung wieder mit der Kochsalz-Lösung des Bestandteils aus Beispiel 2 auf 50 ml aufgefüllt.

Verwendet werden von dem erfindungsgemäßen Wirkstoff (bis zu 18 Substanzen) Wirkstoffe mit einem Molekulargewicht zwischen 5 kDa und 32 kDa.

Für die Anwendung gilt Folgendes:

Man injiziert zunächst 5 bis 10 Tage lang täglich subkutan je nach Schwere des Falles 2 bis 5 mL, dann anschließend jeden 2. Tag 2 bis 3,5 mL des erfindungsgemäßen Wirkstoffes.
Nach aktueller Befundung schleicht man nach frühestens 6 Monaten langsam aus.

## Patentansprüche

1. Pharmazeutischer Wirkstoff zur Behandlung von Hepatitis A, Hepatitis C, Borreliose und/oder Multipler Sklerose, enthaltend in einer pharmazeutisch wirksamen Menge zumindest ungereinigtes Drüsensekret, gewonnen von Tieren der Klasse Diplopoda.

2. Pharmazeutischer Wirkstoff gemäß Anspruch 1, enthaltend in einer pharmazeutisch wirksamen Menge:
a) Drüsensekret eines Diplopoden,
b) zumindest ein Peptidtoxin, und
c) zumindest eine hierzu antagonistisch oder synergistisch wirkende Substanz und/oder eine Durchdringungssubstanz,
wobei das Peptidtoxin aus dem Gift von
• Spinnen, ausgewählt aus der Gruppe, bestehend aus der Familie Loxoscelidae, der Familie Lycosidae und der Familie Dysderidae, oder
• Skorpionen, ausgewählt aus der Gruppe, bestehend aus der Gattung Euscorpius, der Art Euscorpius germanus, den Arten Euscorpius spp. Mallorca I und Mallorca II, der Art Euscorpius balearicus und der Art Euscorpius spp. (Mallorca, Menorca, Ibica)
• Skolopendern der Gattung Scolopendra mit der Artengruppe um die Art cingulata stammt.

3. Pharmazeutischer Wirkstoff gemäß Anspruch 2, wobei die Diplopoden afrikanische Diplopoden sind.

4. Pharmazeutischer Wirkstoff gemäß einem oder mehreren der Ansprüche 2-3, wobei der Wirkstoff zur Behandlung von Borreliose verwendet wird.

5. Pharmazeutischer Wirkstoff gemäß einem oder mehreren der Ansprüche 2-4, **dadurch gekennzeichnet, dass** bei den Spinnen die Gattung Loxosceles, insbesondere Loxosceles rufipes, Loxosceles sp. (Mallorca), Loxosceles sp. (Menorca) und Loxosceles sp. (Ibica), und die Familien Lycosidae und Scytodiae, die Art Dysdera spp. (Mallorca, Menorca, Ibica) und Art Lycosa spp. (Mallorca, Menorca) bevorzugt sind.

6. Pharmazeutischer Wirkstoff gemäß einem oder mehreren der Ansprüche 2-5, **dadurch gekennzeichnet, dass** die antagonistisch oder synergistisch wirkende Substanz und/oder die Durchdringungssubstanz ebenso wie das Peptidtoxin aus einem der in den vorhergehenden Ansprüchen genannten Tieren stammt.

7. Pharmazeutischer Wirkstoff gemäß einem oder mehreren der Ansprüche 2-6, **dadurch gekennzeichnet, dass** die antagonistisch oder synergistisch wirkende Substanz und/oder die Durchdringungssubstanz eine Phospholipase oder eine Hyaluronidase oder eine Kombination beider Substanzen ist.

8. Pharmazeutischer Wirkstoff gemäß einem oder mehreren der Ansprüche 2-7, **dadurch gekennzeichnet, dass** mehrere antagonistisch oder synergistisch wirkende Substanzen und/oder Durchdringungssubstanzen von unterschiedlichen Tieren vorliegen.

9. Pharmazeutischer Wirkstoff gemäß einem oder mehreren der Ansprüche 2-8, **dadurch gekennzeichnet, dass** das Peptidtoxin und/oder die hierzu antagonistisch oder synergistisch wirkende Substanz und/oder die Durchdringungssubstanz durch ein Fraktionierungsverfahren aus dem jeweiligen Gift erhalten wurde.

10. Pharmazeutischer Wirkstoff gemäß Anspruch 9, **dadurch gekennzeichnet, dass** das Fraktionierungsverfahren ausgewählt ist aus der Gruppe, bestehend aus Gelchromatographie, HPLC, Affinitätschromatographie und lonenaustauschchromatographie.

11. Pharmazeutischer Wirkstoff gemäß Anspruch 9, **dadurch gekennzeichnet, dass** der Wirkstoff durch ein Fraktionierungsverfahren unter Verwendung einer Säule mit einem Innendurchmesser von 1,5 cm und einer Höhe von 50 cm, die unten konisch bis auf 1,5 mm zuläuft und die mit 20 ml eines Gleichchromatographiegels AcA 34 mit einer Matrix mit 3% Acrylamid und 4% Agarose, einem Fraktionierbereich von 20 bis 350 kDa, einer Ausschlussgrenze von 750 kDa und einem Kügelchendurchmesser von 60-140 µm gefüllt ist, erhältlich ist, wobei das entsprechende Gift in 0,25 M Tris/HCl, pH 6,5 bis 7,3, und 1,92 M Glycin in destilliertem, deionisiertem Wasser im homogenen Zustand auf das Gel aufgebracht wird und wenn die Giftlösung das Gel durchlaufen hat und 165 ml einer Lösung von 0,25 M Tris/HCl, pH 6,5 bis 7,3, und 1,92 M Glycin in destilliertem, deionisiertem Wasser aufgebracht wurden und die ersten 15 ml des Durchlaufs verworfen und in je 4 ml Fraktionen gesammelt wurden, sich die Peptidtoxine in den Fraktionen 1, 2, 4, 7, 9 und 10 und die antagonistisch oder synergistisch wirkenden Substanzen und/oder die Durchdringungssubstanzen in den Fraktionen 3, 5, 6, 8, 11 und 12 befinden.

12. Pharmazeutischer Wirkstoff gemäß einem oder mehreren der Ansprüch 9-11, **dadurch gekennzeichnet, dass** er ein Peptidtoxin und eine hierzu antagonistisch oder synergistisch wirkende Substanz enthält, die aus verschiedenen Fraktionen des jeweiligen Giftes stammen.

13. Pharmazeutischer Wirkstoff gemäß einem oder mehreren der Ansprüche 2-12, **dadurch gekennzeichnet, dass** er übliche Träger- und Hilfsstoffe und/oder weitere Wirkstoffe enthält.

14. Pharmazeutischer Wirkstoff gemäß Anspruch 13, **dadurch gekennzeichnet, dass** er als übliche Träger- und Hilfsstoffe isotone Lösungen, Eiweißlösungen, Aminosäurelösungen und/oder keimtötende Lösungen, bevorzugt Ringerlösung, 0,9%-ige NaCl-Lösung, Human-Albuminlösung und/oder Glutaminlösung enthält, und/oder dass er als weitere Wirkstoffe Antibiotika, Antimykotika, Antituberkulotika, Mittel gegen Parasiten, Aminosäuren, die Wundbehandlung begünstigende Enzyme, Mitosehemmstoffe und/oder Zytostatika enthält.

15. Pharmazeutischer Wirkstoff gemäß einem oder mehreren der Ansprüche 2-14, **dadurch gekennzeichnet, dass** das Peptidtoxin und/oder die antagonistisch oder synergistisch wirkenden Substanz und/oder die Durchgangssubstanz ein Derivat oder Derivate eines Giftes eines der in Anspruch 1 oder 2 genannten Tieren ist (sind), das (die) in seiner (ihrer) Wirkung dem im Gift der jeweiligen Tieren enthaltenen Toxin entspricht (entsprechen), und das (die) bevorzugt durch chemisch-synthetische Verfahren oder durch rekombinante biotechnologische Methoden gewonnen wird.

16. Verfahren zur Herstellung eines Wirkstoffes nach Anspruch 1, **dadurch gekennzeichnet, dass** den zu melkenden Tieren Drüsensekret an den Beinen abgenommen und gefriergetrocknet wird.

17. Verfahren gemäß Anspruch 16, **dadurch gekennzeichnet, dass** das gefriergetrocknete Sekret mit 0,9-%iger NaCl-Lösung vermengt wird.

18. Verfahren gemäß Anspruch 16 oder 17, **dadurch gekennzeichnet, dass** die Gewinnung des Drüsensekrets bei Reinraumbedingungen bei einer Temperatur von 25°C bis 28°C vorgenommen wird.

19. Verfahren gemäß Anspruch 16 bis 18, **dadurch gekennzeichnet, dass** 1 mg Drüsensekret mit 500 ml NaCl-Lösung vermengt wird.

20. Verfahren zur Herstellung eines Medikaments zur Behandlung von Borreliose, **dadurch gekennzeichnet, dass** der Wirkstoff gemäß Anspruch 1 in einer Konzentration von 1 mg Drüsensekret auf 500 ml NaCl-Lösung vorliegt.

21. Verfahren zur Herstellung eines Wirkstoffes nach einem der Ansprüche 2-15 mit den Schritten:
a) Lösen von Drüsensekret eines afrikanischen Diplopoden in Kochsalzlösung mit 10% eines Peptidtoxids und einer hierzu antagonistisch oder synergistisch wirkende Substanz und/oder einer Durchdringungssubstanz bis zur Sättigung;
b) Erwärmen der so erhaltenen Lösung auf 50°C über mindestens 3 Tage in einem sterilen Abzug;
c) Auffüllen (auf Marke) der Lösung mit einer Kochsalzlösung mit 10% eines Peptidtoxids und einer hierzu antagonistisch oder synergistisch wirkende Substanz und/oder einer Durchdringungssubstanz.
wobei das Peptidtoxin aus dem Gift von
• Spinnen, ausgewählt aus der Gruppe, bestehend aus der Familie Loxoscelidae, der Familie Lycosidae, der Familie Dysderidae und Lycosa spp. (Mallorca, Menorca), oder
• Skorpionen, ausgewählt aus der Gruppe, bestehend aus der Gattung Euscorpius, der Art Euscorpius germanus, den Arten Euscorpius spp. Mallorca I und Mallorca II, der Art Euscorpius Balearicus und der Art Euscorpius spp. (Mallorca, Menorca, Ibica)
• Skolopendern der Gattung Scolopendra mit der Art cingulata stammt.

22. Verfahren gemäß Anspruch 21, wobei das Gift aus weiblichen Tieren gewonnen wird.

23. Verfahren gemäß Anspruch 21 oder 22, **dadurch gekennzeichnet, dass** das Gift durch manuelles Melken gewonnen wird.

24. Verfahren gemäß einem oder mehreren der Ansprüche 21-23, **dadurch gekennzeichnet, dass** das Peptidtoxin und/oder die hierzu antagonistisch oder synergistisch wirkende Substanz und/oder die Durchdringungssubstanz durch ein Fraktionierungsverfahren aus dem jeweiligen Gift erhalten wurde, und das Gift bevorzugt vor der Fraktionierung homogenisiert wird.
